# EUROPÄISCHE PATENTSCHRIFT

(11) **EP 0 094 541 B1**
(45) Veröffentlichungstag und Bekanntmachung des Hinweises auf die Patenterteilung: **14.01.1987**
(21) Anmeldenummer: 83104272.6
(22) Anmeldetag: 02.05.1983
(51) Int. Cl.: C07D 285/12, A01N 43/82, C07D 417/12

(54) **Substituierte 5-Trifluormethyl-1,3,4-thiadiazol-2-yl-oxyessigsäureamide, Verfahren zu ihrer Herstellung und ihre Verwendung als Herbizide**
Substituted 5-trifluoromethyl-1,3,4-thiadiazol-2-yl oxiacetic-acid amides, process for their preparation and their use as herbicides
Amides substituées de l'acide 5-trifluoro-méthyl-1,3,4 thiadiazol-2-yl-oxyacétique; leur procédé de préparation et leur application comme herbicides

(30) Priorität: 15.05.1982 DE 3218482
(43) Veröffentlichungstag der Anmeldung: 23.11.1983
(73) Patentinhaber: BAYER AG, 51368 Leverkusen (DE)
(72) Erfinder: Förster, Heinz, Dr., D-5600 Wuppertal 1 (DE); Hofer, Wolfgang, Dr., verstorben (DE); Schmidt, Robert R., Dr., D-5060 Bergisch-Gladbach 2 (DE); Eue, Ludwig, Dr., D-5090 Leverkusen 1 (DE)

## Beschreibung

Die Erfindung betrifft neue substituierte 5-Trifluor-methyl-1,3,4-thiadiazol-2-yl-oxyessigsäureamide, ein Verfahren zu ihrer Herstellung und ihre Verwendung als Herbizide.

Es ist bereits bekannt, daß bestimmte Azolyloxycarbonsäureamide als Herbizide verwendet werden können (vgl. z.B. DE-OS 29 14 003 und DE-OS 30 04 326). So kann z.B. 4,5-Dichlor-1,3-thiazol-2-yl-cxyessigsäure-N,N-diethylamid zur selektiven Bekämpfung von Gräsern in dikotylen Kulturpflanzen, wie z.B. Baumwolle, eingesetzt werden; gegen dikotyle Unkräuter ist die Verbindung jedoch nicht ausreichend wirksam.

Es wurden nun neue substituierte 5-Trifluormethyl-1,3,4-thiadiazol-2-yl-oxyessigsäureamide der allgemeinen Formel 1 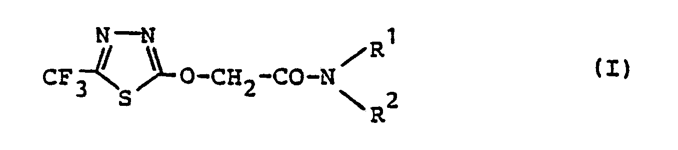 aufgefunden, in welcher
R^{l} und R2, welche gleich oder verschieden sein können, einzeln für Alkyl, Cyanoalkyl, Alkoxyalkyl, Alkylthioalkyl, Alkenyl, Alkinyl, Alkoxy, jeweils mit bis zu 10 C-Atomen, Cycloalkyl oder Cycloalkenyl mit jeweils bis zu 12 C-Atomen, Aralkyl mit 1 oder 2 Kohlenstoffatomen im Alkylteil und 6 oder 10 Kohlenstoffatomen im Arylteil, welcher gegebenenfalls durch Halogen substituiert ist, oder für Aryl mit 6 oder 10 Kohlenstoffatomen stehen, wobei der Arylrest durch 1 bis 3 Halogenatome, 1 bis 3 Alkylgruppen mit jeweils 1 bis 4 Kohlenstoffatomen, Nitro, Cyano oder Alkoxy mit 1 bis 4 Kohlenstoffatomen substituiert sein kann, oder worin die Reste R^{l} und R² zusammen mit dem Stickstoffatom, an das sie gebunden sind, einen gegebenenfalls durch 1 bis 3 Alkylgruppen mit jeweils 1 bis 5 Kohlenstoffatomen substituierten, gegebenenfalls teilweise ungesättigten und/oder benzannellierten Monocyclus oder Bicyclus mit bis zu 15 Kohlenstoffatomen bilden, worin die Reste R^{l} und R² zusammen mit dem Stickstoffatom, an das sie gebunden sind, einen gegebenenfalls durch 1 bis 3 Alkylgruppen mit jeweils 1 bis 5 Kohlenstoffatomen substituierten, gesättigten und ein weiteres Stickstoffatom, Sauerstoffatom oder Schwefelatom enthaltenden Monocyclus mit 1 bis 5 zu Kohlenstoffatomen bilden - mit der Maßgabe, daß nicht gleichzeitig R^{l} für Methyl und R² für Phenyl stehen.

Man erhält die neuen Verbindungen der allgemeinen Formel I, wenn man Hydroxyessigsäureamide der allgemeinen Formel II 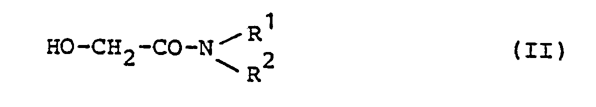 in welcher
R^{l} und R die oben angegebene Bedeutung haben,
mit 2-Chlor-5-trifluormethyl-1,3,4-thiadiazol der Formel 111 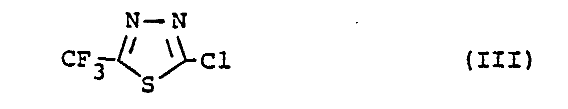
in Gegenwart eines Säureakzeptors und gegebenenfalls unter Verwendung eines Verdünnungsmittels, umsetzt..

Die neuen substituierten 5-Trifluormethyl-1,3,4-thia-diazol-2-yl-oxyessigsäureamide der allgemeinen Formel zeichnen sich durch starke herbizide Wirksamkeit aus.

Überraschenderweise sind die erfindungsgemäßen Wirkstoffe der allgemeinen Formel außer gegen Gräser auch gegen dikotyle Unkräuter sehr wirksam. Die neuen Wirkstoffe haben somit wesentliche Vorteile gegenüber den vorbekannten Azolyloxycarbonsäureamiden (gemäß DE-OS 29 14 003 und 30 04 325), welche im wesentlichen nur gegen Gräser wirken.

Gegenstand, der Erfindung sind vorzugsweise 5-Trifluor-methyl-1,3,4-thiadiazol-2-yl-oxyessigsäureamide der allgemeinen Formel in welcher
R¹ für C₁-C₅-Alkyl, Cyanoethyl, Cₗ-C₄-Alkoxy-ethyl,
Allyl, propargyl, 1-Methyl-propargyl oder 1,1-Di-methyl-propargyl steht und
R² für C₁-C₅-Alkyl, C₁-C₅-Alkoxy, Cyanoethyl, Cₗ-C₄-Alkoxy-ethyl, Allyl, Propargyl, 1-Methyl-propargyl, 1,1-Dimethylpropargyl, Cyclopentyl, Cyclohexyl, 3,4,6-Trimethyl-cyclohexen-1-yl, Benzyl, Naphthyl oder Phenyl, welches gegebenenfalls durch 1 bis 3 Reste (Methyl, Chlor, Fluor, Trifluormethyl, Methoxy, Methylthio, Trifluormethoxy und/oder Trifluormethylthio) substituiert ist, steht
oder worin die Reste R^{I} und R² zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, für Perhydroazepinyl (Hexamethylenimino-Rest), für den Heptamethylen imino-Rest, für 1,2,3,4-Tetrahydroindolyl, für Monoalkyl oder Dialkyltetrahydroindolyl mit bis zu 3 Kohlenstoff atomen je Alkylgruppe, für Perhydroindolyl, Monoalkyloder Dialkylperhydroindolyl mit 1 bis 3 Kohlenstoffatomen je Alkylgruppe, für 1,2,3,4-Tetrahydrochinolyl oder 1,2,3,4-Tetrahydro-iso-chinolyl, Monoalkyl- oder Di-alkyl-1,2,3,4-tetrahydrochinolyl oder -iso-chinolyl mit 1 bis 3 Kohlenstoffatomen je Alkylgruppe, für perhydrochinolyl oder Perhydro-iso-chinolyl, Monoalkyl- oder Dialkylperhydrochinolyl oder -perhydroisochinolyl mit 1 bis 3 Kohlenstoffatomen je Alkylgruppe, für perhydrothiazolyl, für Perhydrooxazolyl, für Perhydrooxazinyl oder für den Rest 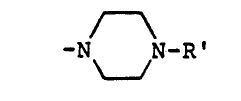
stehen, worin R' für Cₗ-C₄-Alkyl, für gegebenenfalls durch C₁-C₂-Alkyl, Cₗ-C₂-Alkoxy, Fluor, Chlor, Brom, Trifluormethyl und/oder Nitro substituiertes Phenyl steht, - wiederum mit der Maßgabe, daß nicht gleichzeitig Rl für Methyl und R² für Phenyl steht.

Verwendet man 2-Chlor-5-trifluormethyl-1,3,4-thiadiazol und beispielsweise Hydroxyessigsäure-2-ethylpiperidid als Ausgangsstoffe, so kann der Reaktionsablauf nach dem erfindungsgemäßen Verfahren durch folgendes Formelschema wiedergegeben werden:

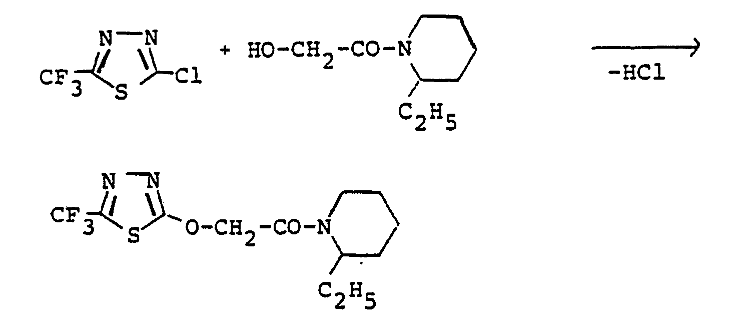

Die als Ausgangsstoffe zu verwendenden Hydroxyessigsäureamide sind durch die allgemeine Formel 11 definiert. In dieser Formel stehen R¹ und R² vorzugsweise bzw. insbesondere für diejenigen Reste, welche bereits im Rahmen der Substituentendefinitionen der allgemeinen Formel I vorzugsweise bzw. als insbesondere bevorzugt genannt sind.

Als Ausgangsstoffe der allgemeinen Formel II seien beispielsweise genannt:
Hydroxyessigsäure-dimethylamid, -diethylamid, -di-n-propylamid, -di-iso-propylamid, -N-methyl-N-iso-propylamid, -N-methyl-N-isobutyl-amid, -N-methyl-N-sek.-butylamid, -N-propyl-N-sek.-butyl-amid, -N-methyl-N-(2-cyano-ethyl)-amid, -di-(2-methoxy-ethyl)-amid, -di-allyl-amid, -N-methyl-N-propargyl-amid, -N-methyt-N-(1-methyl-pro-pargyl)-amid, -dipropargyl-amid, -N-methyl-N-cyclopentylamid, -N-methyl-N-cyclohexyt-amid, -N-methyl-N-(2-nitro-phenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phe-nyl)-amid, -N-methyl-N-13-nitro-6-methyl-phenyl)-amid, -N-ethyl-anilid, -N-ethyl-N-(2-nitro-phenyl)-, -N-ethyl-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-8-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlorphenyl)-, -N-propyl-N-(4-chlor-phenyl)-amid, -N-propyl-N-(2-methyl-phenyl)-, -N-pro-pyl-N-(3-methyl-phenyl)-, -N-propyl-N-(4-methyl-phenyl)-amid, -N-propyl-N-(3-nitro-6-methyl-phenyl)-amid, -Nbutyl-anilid, -N-methyl-N-naphth-(1)-ylamid, -N-methyl-N-naphth-(2)-ylamid, -N-ethyl-N-naphth-(1)-ylamid, -N-ethyl-N-naphth-(2)-ylamid, -N-methyl-N-benzylamid, -N-ethyl-N-benzylamid, -N-propyl-N-benzylamid, -N-butyl-N-benzylamid, -pyrrolidid, -1-methyl-pyrrolidid, -morpholid, -piperidid, -2-methyl-piperidid, -2-methyl-piperidid, -4-methyl- piperidid, -2,4-dimethyl-piperidid, -3,5-di-methyl-piperidid, -3,5-diethylpiperidid, -2,4,6-tri-methyl-piperidid, -2-ethyl-piperidid, -4-ethyt-piperidid, -2,4-diethyl-piperidid, -2,4,6-triethyl-piperidid, -2-methyl-4-ethyl-piperidid, -2 -ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, 2,2-dimethyl-perhydroindolid, -2-methyl-1,2,3,4-tetrahydrochinolid, -perhydrochinolid, -2-methyl- perhydrochinolid, -1,2,3,4-tetrahydro-isochinolid und -perhydroisochinolid; ferner -N-methyl-N-(2-methylthiophenyl)-, -N-methyl-N-(3-methylthio-phenyl)-und -N-methyl-N-(4-methylthio-phenyl)-amid; -N-methyl-N-(2- fluorphenyl)-, -N-methyl-N-(3-fluorphenyl)- und -N-methyl-N-(4-fluorphenyl)-amid; -N-methyl-N-(2-trifluormethylphenyl)-, -N-methyl-N-(3-trifluormethylphenyl)- und -N-methyl-N-(4-trifluormethylphenyl)-amid; -N-methyl-N-(2-trifluormethoxyphenyl)-, -N-methyl-N-(3-trifluor-methoxyphenyl)- und -N-methyl-N-(4-trifluormethoxyphenyl)amid; -N-methyl-N-(2-trifluorethylphenyl)-amid.

Die Hydroxy-carbonsäureamide der allgemeinen Formel 11 sind teilweise bekannt (vgl. US-PS 3 399 988; DE-OS'en 2 201 432 und 2 647 481). Sie können, wie im nachstehenden Schema skizziert ausgehend von Chloracetylchlorid hergestellt werden:

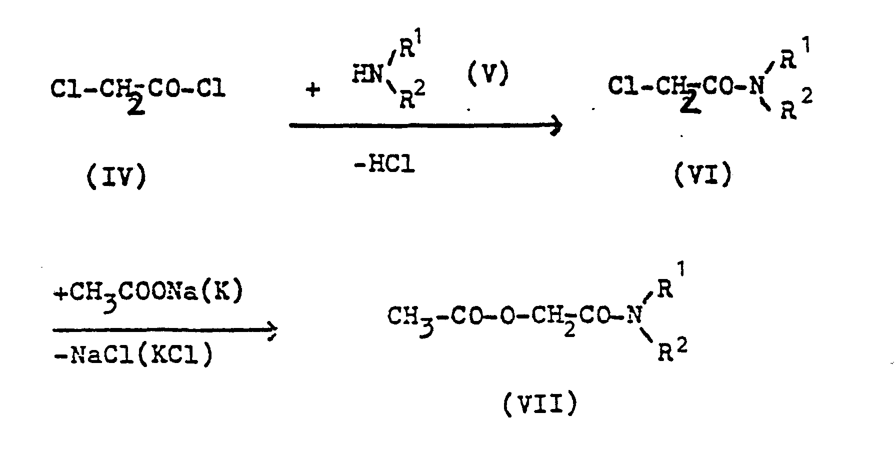

Hierzu wird zunächst das literaturbekannte Chloracetylchlorid der Formel (IV) mit Aminen der allgemeinen Formel V - wobei R^{l} und R² die oben angegebene Bedeutung haben - gegebenenfalls in Gegenwart eines 8äurebindemittels wie z.B. Triethylamin und gegebenenfalls unter Verwendung eines inerten Verdünnungsmittels wie z.B. 1,2-Dichlorethan, bei Temperaturen zwischen -20 und 100°C, vorzugsweise zwischen -10 und 50°C in die entsprechenden Chloressigsäureamide der allgemeine Formel VI überführt. Die Aufarbeitung dieser produkte erfolgt nach üblichen Methoden durch Waschen mit Wasser, Trocknen der organischen Phase und Abdestillieren des Lösungsmittels.

Die Verbindungen der allgemeinen Formel VI werden mit Natrium- oder Kalium-acetat, gegebenenfalls unter Verwendung eines Verdünnungsmittels wie z.B. Essigsäure oder Dimethylsulfoxid, bei Temperaturen zwischen 20 und 150°C, vorzugsweise zwischen 50 und 120°C, zu den entsprechenden Acetoxy-essigsäureamiden der allgemeinen Formel VII umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wäßrig-alkoholischer Natronlauge oder Kalilauge bei Temperaturen zwischen 0 und 100°C, vo-r-zugsweise zwischen 10 und 50°C, können die Verbindungen der allgemeinen Formel VII) zu den Verbindungen der allgemeinen Formel 11 entacyliert werden. Zur. Isolierung der Produkte werden die Lösungsmittel im Vakuum abdestilliert, der Rückstand mit einem organischen Lösungsmittel wie z.B. Methylenchlorid oder Essigester extrahiert, die Lösung getrocknet und das Lösungsmittel abdestilliert.

Das als Ausgangsstoff zu verwendete 2-Chlor-5-trifluor-methyl-1,3,4-thiadiazol ist durch die Formel 111 definiert. Diese Verbindung ist literaturbekannt [vgl. J. Heterocyclic Chem. 1974, Vol. 11(3), S. 343-345]; sie kann hergestellt werden, beispielsweise indem man die entsprechende 2-Amino-Verbindung mit Salzsäure in Wasser löst und unter Eiskühlung mit Natriumnitrit umsetzt, die Mischung einige Stunden bei Temperaturen zwischen -10 und +50° C rührt, das Reaktionsprodukt mit Toluol extrahiert und nach Waschen und Trocknen die organische Phase destillativ aufarbeitet.

Das Verfahren zur Herstellung der neuen Verbindungen der allgemeinen Formel I wird vorzugsweise unter Verwendung geeigneter Lösungs- oder Verdünnungsmittel durchgeführt. Als solche kommen praktisch alle inerten organischen Solventien in Frage. Hierzu gehören insbesondere Alkohole, wie Methanol, Ethanol, n- und iso-Propanol, n-, iso-, sek.- und tert.-Butanol, Ether wie Diethylether, Dibutylether, Tetrahydrofuran, Diglyme und Dioxan, Ketone wie Aceton, Methylethylketon, Methylisopropylketon und Methylisobutylketon, Nitrile wie Acetonitril und Propionsäurenitril, sowie die hochpolaren Lösungsmittel Dimethylfornmamid, Dimethylsulfoxid, Sulfolan und Hexamethylphosphorsäuretriamid.

Als Säureakzeptoren können praktisch alle üblicherweise verwendbaren Säurebindemittel eingesetzt werden: hierzu gehören insbesondere Alkali- und Erdalkalihydroxide bzw. -oxide wie Natrium-, Kaliumhydroxid und insbesondere Lithiumhydroxid sowie Calciumoxid oder Calcium-hydroxid, Alkali- und Erdalkali-carbonate wie Natrium-, Kaliumund Calciumcarbonat, Alkalialkoholate wie Natrium-methylat, -ethylat und -tert-butylat, Kaliummethylat, -ethylat und -tert.-butylat, ferner aliphatische, aromatische oder heterocyclische Amine wie Triethylamin, Dimethylanilin, Dimethylbenzylamin, Pyridin, Diazabicyclooctan und Diazabicycloundecen.

Die Reaktionstemperatur kann innerhalb eines größeren Bereichs variiert werden. Im allgemeinen arbeitet man zwischen -50 und +150°C, vorzugsweise bei -20 bis +100°C.

Das erfindungsgemäße Verfahren wird im allgemeinen bei Normaldruck durchgeführt.

Bei der Durchführung des erfindungsgemäßen Verfahrens setzt man auf 1 Mol 2-Chlor-5-trifluormethyl-1,3,4-thiadiazol der Formel 111 1,0 bis 1,5 Mol Hydroxyessigsäureamid der allgemeinen Formel 11 ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden: Man destilliert gegebenenfalls einen Teil des Verdünnungsmittels unter vermindertem Druck ab und gießt den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel wie z.B. Toluol oder Methylenchlorid extrahiert; nach Waschen und Trocknen wird dann von der organischen Phase das Lösungsmittel im Vakuum abdestilliert. Die zurückleibenden Produkte werden durch ihren Schmelzpunkt bzw. ihren Brechungsindex charakterisiert.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrieund Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gunmi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten Wirkung gegen monokotyle Unkräuter auch eine gute herbizide Wirkung bei dikotylen Unkräutern. Ein selektiver Einsatz der erfindungsgemäßen Wirkstoffe ist in verschiedenen Kulturen möglich, vor allem in dikotylen Kulturpflanzen sowie in Reis und Getreide..

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermitrel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-%. Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln, ist möglich.

Die Wirkstoffe können als solche, in Form ihrer Formulierungen oder der daraus durch weiteres Verdünnen bereiteten Anvendungsformen, wie gebrauchsfertige Lösungen, Suspensionen, Emulisionen, Pulver, Pasten und Granulate angewandt werden. Die Anwendung geschieht in üblicher Weise, z.B. durch Geißen, Spritzen, Sprühen, Streuen oder Stäuben.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfaheren, vorgenommen Sie können auch vor der Saat in den Boden eingearbeitet werden.

Die aufgewandte Wirksoffmenge kann in größeren Bereichen schwanken. Sie hängt im wesentlichen von der Art des gewünschten Effektes ab. Im ailgemeinen liegen die Aufwandmengen zwischen 0,01 und 10 kg Wirkstoff pro ha, vorzugsweise zwischen 0,1 und 5 kg/ha.

Die nachfolgenden Beispiele dienen zur weiteren Erläuterung der Erfindung.

### Herstellungsbeispiele

### Beiseiel 1

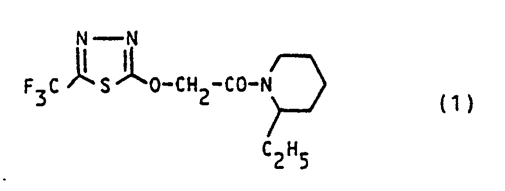

Zu einer Lösung von 2,8 g (0,025 Mol) Kalium-tert.-butanolat in 50 ml tert.-Butanol werden bei einer Temperatur zwischen 20 und 30° C nacheinander 4,3 g (0,025 Mol) Hy-droxyessigsäure-2-ethyl-piperidid und 4,7 g (0,025 Mol) 2-Chlor-5-trifluormethyl-1,3,4-thiadiazol gegeben. Das Reaktionsgemisch wird 3 Stunden gerührt, dann mit 100 ml Methylenchlorid verdünnt, mit 50 ml 2n Salzsäure und dann mit 50 ml Wasser gewaschen, getrocknet und durch Einengen im Wasserstrahlvakuum vom Lösungsmittel befreit.

Man erhält 5,0 g (66% der Theorie) (5-Triflluormethyl-1,3,4-thiadiazol-2-yl-oxy)-essigsäure-2-ethylpiperdid als _{Öl} vom Brechungsindex 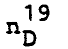 = 1 4954.

### Beispiel 2

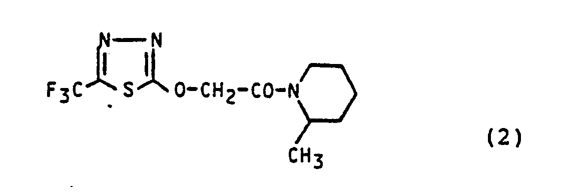

Eine Lösung von 2,4 g (0,06 Mol) Natriumhhydroxid in 5 ml wasser wird zu einer auf-20°C gekühlten Lösung von 12,0 (0,06 Mol) 2-Chlor-5-trifluormethyl-1,3,4-thiadiazol und 10,0 g Hydroxyessigsäure-2-methylpiperidid in 100 ml Toluol tropfenweise gegeben.

Das Reaktionsgemisch wird 2 Stunden bei -20°C gerührt, in 100 ml Wasser gegossen und mit 2n Salzsäure angesäuert. Die organische Phase wird abgetrennt, mit Wasser gewaschen, getrocknet und durch Einengen im Wasserstrahlvakuum vom Lösungsmittel befreit.

Man erhält 10,0 g (54 % der Theorie) 5-Trifluormethyl-1,3,4-thiadiazol-2-yl-oxy)-essigsäure-2-methylpiperidid als Öl vom Brechungsindex n_{D}²⁰ = 1,4934.

Analog den Beispielen 1 und 2 können die in der nachstehenden Tabelle aufgeführten Verbindungen der allgemienen Formel I hergestellt werden: 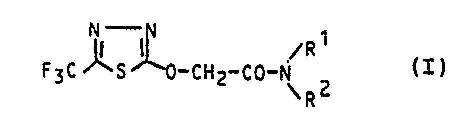 Tabelle 1 Beispiele für Verbindungen der allgemeien Formel

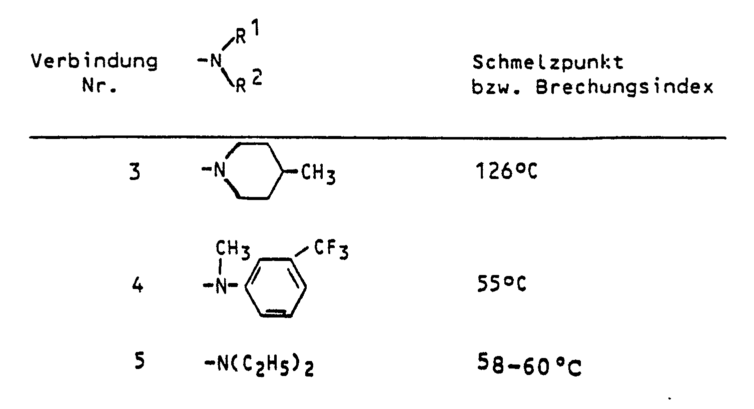

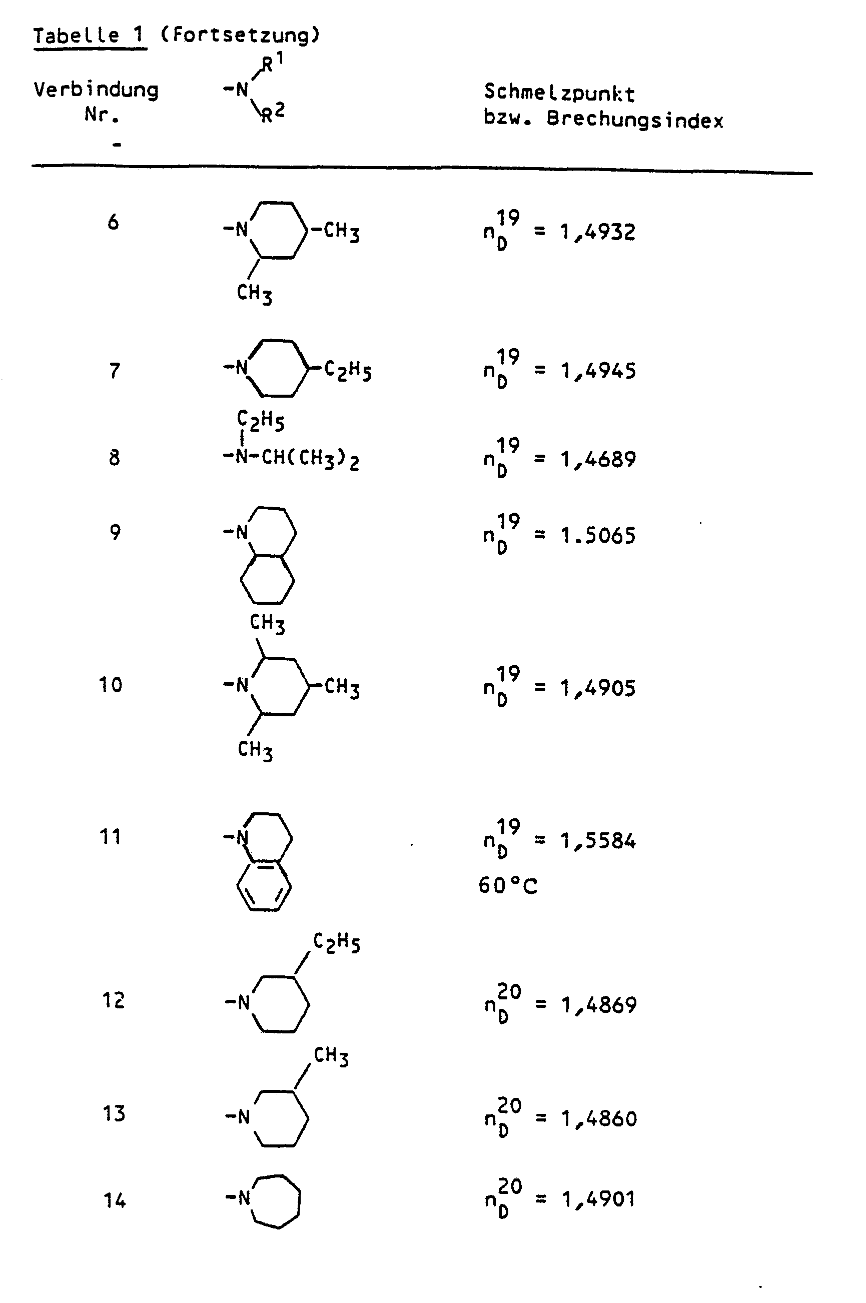

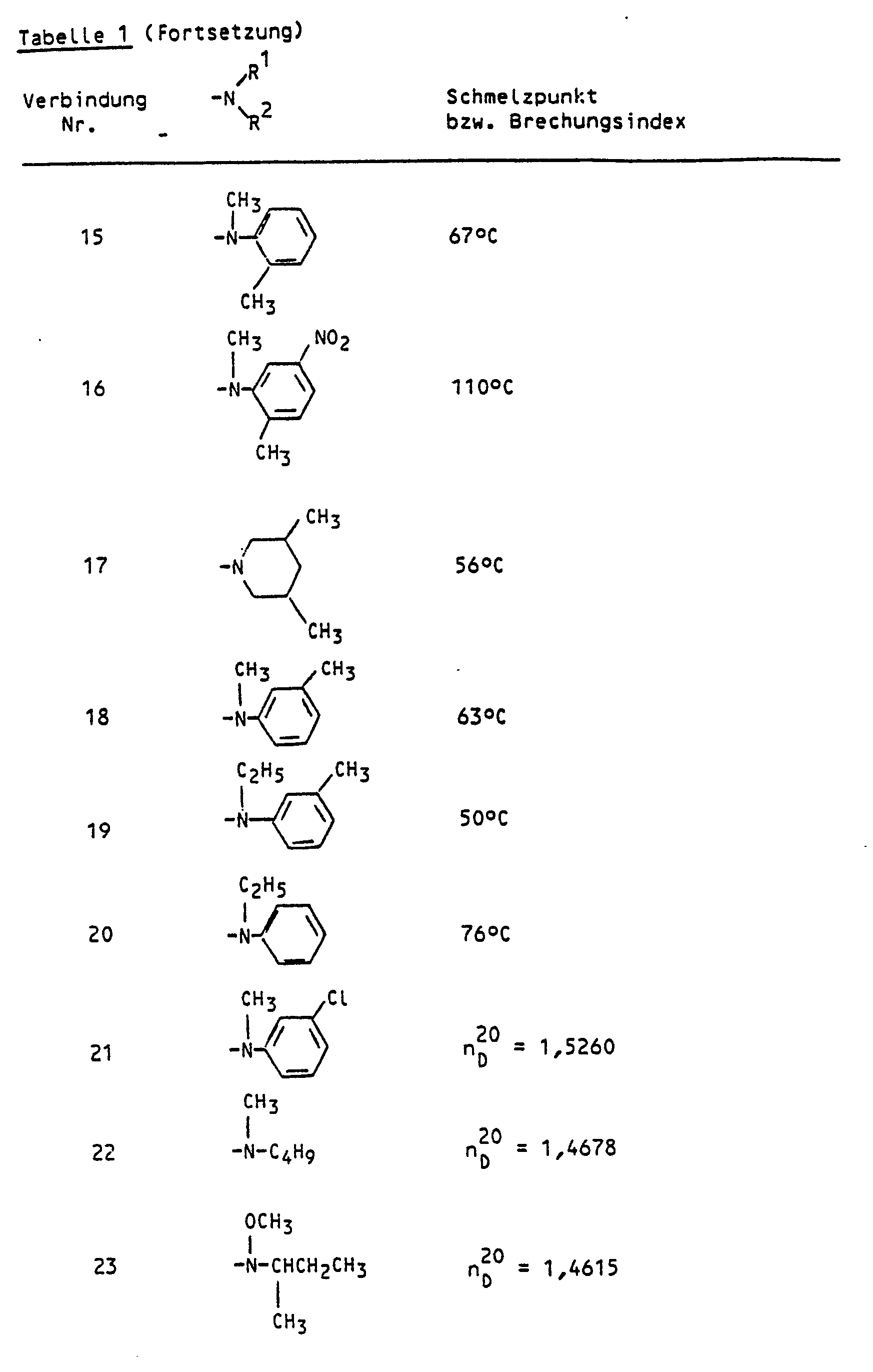

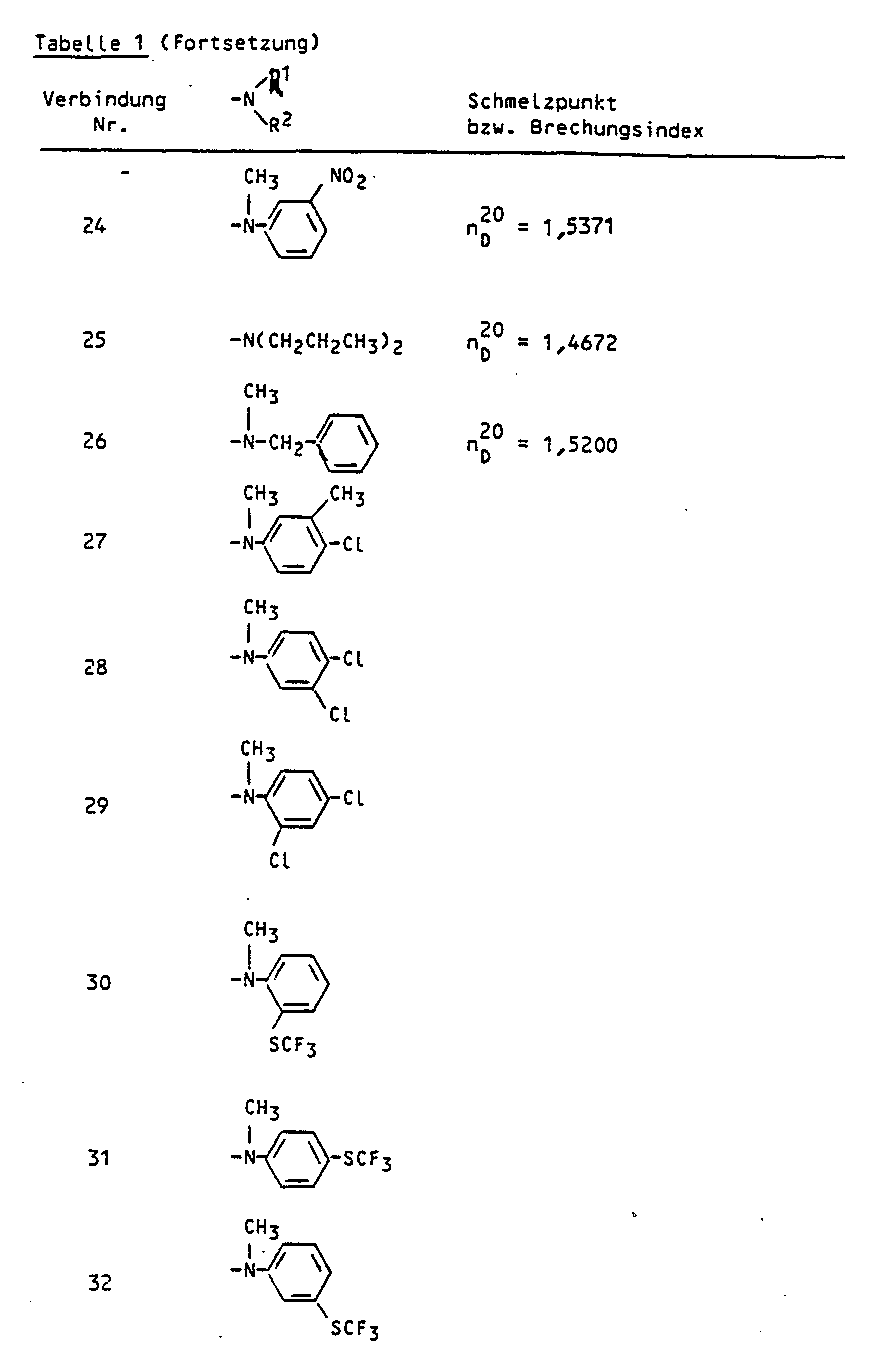

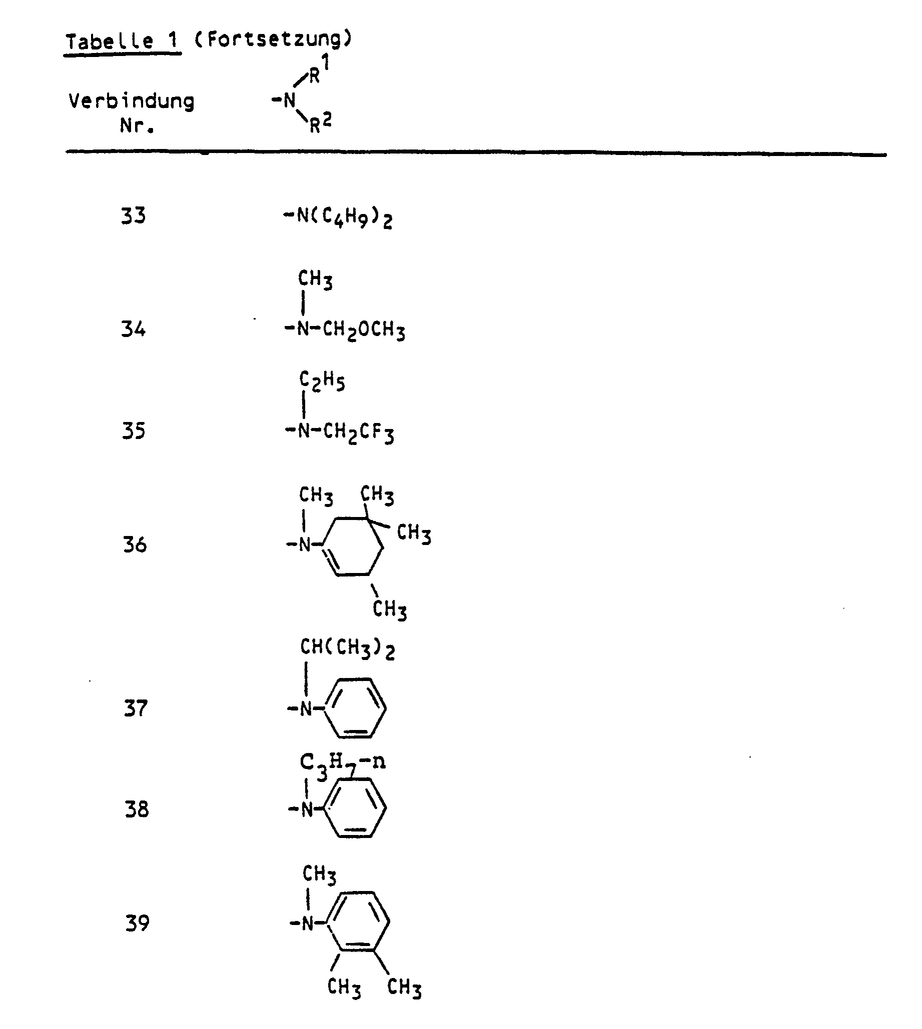

Das als Ausgangsverbindung einzusetzende 2-Chlor-5-tri-fluoromethyl -1,3,4-thiadiazol kann wie folgt hergestellt werden:

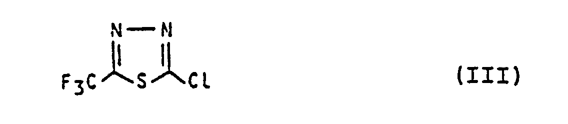

Eine Mischung aus 126 g (0,75 Mol) 2-Amino-5-trifluor-methyl-1,3,4-thiadiazol, 17,9 g (0,28 Mol) Kupferpulver und 1,5 1 konz. Salzsäure wird auf -10°C gekühlt und tropfenweise mit einer Lösung von 207 g (3 Mol) Natriumnitrit in 300 ml Wasser versetzt. Das Gemisch wird eine Stunde bei 0°C gerührt und eine Stunde auf 40 bis 50°C erwärmt. Man extrahiert mit 500 ml Methylenchlorid, wäscht die organische Lösung zweimal mit je 300 ml Wasser, trocknet über Natriumsulfat, filtriert, destilliert bei Normaldruck das Lösungsmittel ab und destilliert den Rückstand im Wasserstrahlvakuum.

Man erhält 90 g (64 % der Theorie) 2-Chlor-5-trifluor-methyl-1,3,4-thiadiazol vom Siedepunkt 46°C (16 mbar).

### Beispiel A

### Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen z.B. die folgenden Verbindungen gemäß Herstellungsbeispielen eine ausgezeichnete Wirksamkeit:1, 2, 5,15,18.

## Patentansprüche

1. Substituierte 5-Triflourmethyl-1,3,4-thiadiazol-2-yl-oxyessigsäureamide der allgemeinen Formel 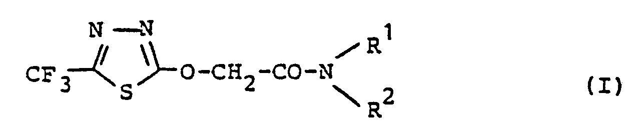 in welcher
R¹ und R² gleich oder verschieden sind und einzeln für Alkyl, Cyanoalkyl, Alkoxyalkyl, Alkylthioalkyl, Alkenyl, Alkinyl, Alkoxy, jeweils mit bis zu 10 C-Atomen, Cycloalkyl oder Cycloalkenyl mit jeweils bis zu 12 C-Atomen, Aralkyl mit 1 oder 2 Kohlenstoffatomen im Alkylteil und 6 oder 10 Kohlenstoffatomen im Arylteil, welcher gegebenenfalls durch Halogen substituiert ist, oder für Aryl mit 6 oder 10 Kohlenstoffatomen stehen, wobei der Arylrest durch 1 bis 3 Hologenatome, 1 bis 3 Alkylgruppen mit jeweils 1 bis 4 Kohlenstoffatomen, Nitro, Cyano oder Alkoxy mit 1 bis 4 Kohlenstoffatomen substituiert sein kann, oder worin die Reste R¹ und R² zusammen mit dem Stickstoffatom, an das sie gebunden sind, einen gegebenenfalls durch 1 bis 3 Alkylgruppen mit jeweils 1 bis 5 Kohlenstoffatomen substituierten, teilweise ungesättigten und/oder benzannellierten Monocyclus oder Bicyclus mit bis zu 15 Kohlenstoffatomen bilden, oder worin die Reste R¹ und R² zusammen mit dem Stickstoffatom, an das sie gebunden sind, einen gegebenenfalls durch 1 bis 3 Alkylgruppen mit jeweils 1 bis 5 Kohlenstoffatomen substituierten, gesättigten und ein weiteres Stickstoffatom, Sauerstoffatom oder Schwefelatom enthaltenden Monocyclus mit 1 bis zu 5 Kohlenstoffatomen bilden - mit der Maßgabe, daß nicht gleichzeitig R¹ für Methyl und R² für Phenyl stehen.

2. (5-Trifluormethyf-1,3,4-thiadiazol-2-yl-oxy)-essi säure-2-methylpiperidid der Formel 2 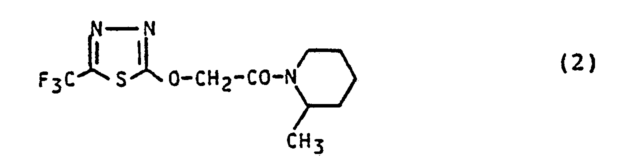 gemäß Anspruch 1.

3. (5-Triflourmethyl-1,3,4-thiadiazol-2-yl-oxy)-essig-säure-2-ethylpiperidid der Formel 1 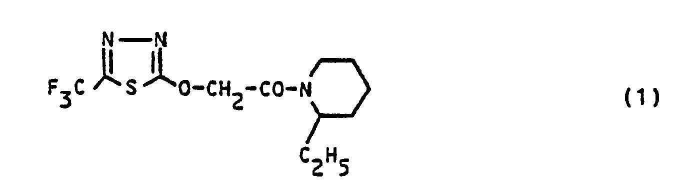 gemäß Anspruch 1.

4. (5-Trifluormethyl-1,3,4-thiadiazol-2-yl-oxy)-essigsäure-N-methyl-N-(2-methyl-phenyl)-amid der Formel 15 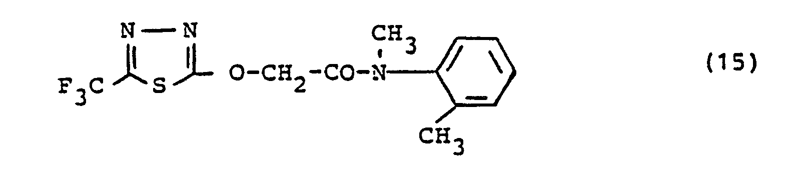 gemäß Anspruch 1.

5. (5-Trifluormethyl-1,3,4-thiadiazol-2-yl-oxy)-essig säure-N-methyl-N-(3-methyl-phenyl)-amid der Formel 18 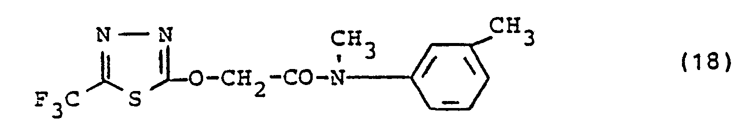 gemäß Anspruch 1.

6. (5- Trifluormethyl-1,3,4-thiadiazol-2-yl-oxy)-essigsäure-N,N-diethylamid der Formel 5 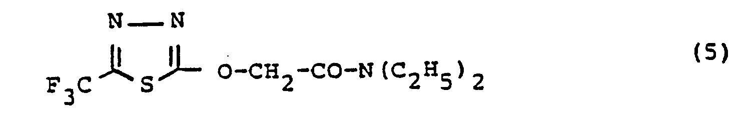 gemäß Anspruch 1

7. (5-Trifluormethyl-1,3,4-thiadiazol-2-yl-oxy)-essigsäure-N, N(di-n-propylamid)der Formel 25 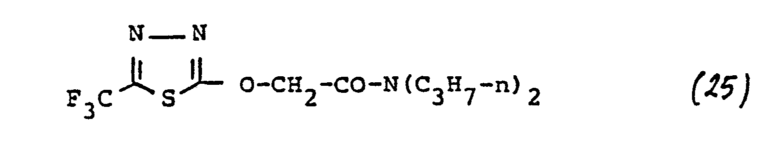 gemäß Amspruch 1

8. Verfahren zur Herstellung von substituierten 5-Tri-fluormethyl-1,3,4-thiadiazol-2-yl-oxyessigsäureamiden der allgemeinen Formel gemäß Anspruch 1, dadurch gekennzeichnet, daß man Hydroxyessigsäureamide der allgemeinen Formel II 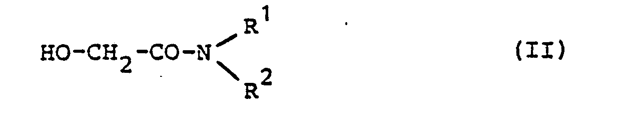 in welcher
Rₗ und R₂ die oben angegebene Bedeutung haben, mit 2-Chlor-S-trifluormethyl-1,3,4-thiadiazol der Formel III 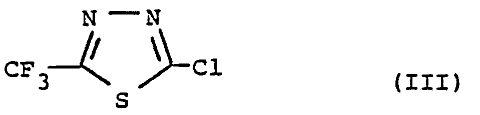 in Gegenwart eines Säureakzeptors und gegebenenfalls unter Verwendung eines Verdünnungsmittels umsetzt.

9. Herbizide Mittel, gekennzeichnet durch einen Gehalt an substituierten 5-Trifluormethyl-1,3,4-thiadiazol-2-yl-oxyessigsaureamiden der allgemeinen Formel gemäß Anspruch 1.

10. Verwendung von substituierten 5-Trifluormethyl-1,3,4-thiadiazol-2-yl-oxyessigsäureamiden der allgemeinen Formel I gemäß Anspruch 1 zur Bekämpfung von Pflanzenwachstum, .

11. Verfahren zur Herstellung von herbiziden Mitteln, dadurch gekennzeichnet, da. man substituierte 5-Triflourmethyl-1,3,4-thiadiazol-2-yl-oxyssigsäure-amide der allgemeinen Formel I gemäß Anspruch 1 mit Streckmitteln und/ oder oberflächenaktiven Mitteln vermischt.

## Claims

1. Substitueted 5-trifluoromethyl-1,3,4-thiadiazol 2-yl-oxyacetic acid amides of the general formula I 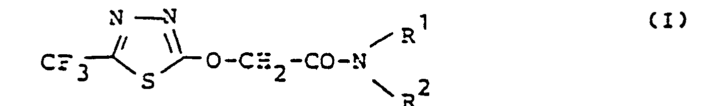 in which
R^{l} and R² are identical or different and each represent alkyl, cyanoalkyl, alkoxyalkyl, alkylthioalkyl, alkenyl, alkinyl or alkoxy, each having up to 10 C atoms, cycloalkyl or cyclo- alkenyl, each having up to 12 C atoms, or aralkyl which has 1 or 2 carbon atoms in the alkyl part and 6 or 10 carbon atoms in the aryl part which is optionally substituted by halogen, or aryl which has 6 or 10 carbon atoms, it being possible for the aryl radical to be substituted by 1 to 3 halogen atoms, 1 to 3 alkyl groups each having i to 4 carbon atoms, nitro, cyano or alkoxy having 1 to 4 carbon atoms,
or wherein
the radicals R¹ and R², together with the nitrogen atom to which they are bonded, form an optionally partially unsaturated and/or benzo-fused monocyclic or bicyclic structure which has up to 15 carbon atoms and is optionally substituted by 1 to 3 alkyl groups, each having 1 to 5 carbon atoms,
or wherein
the radicals R^{l} and R², together with the nitrogen atom to which they are bonded, form a saturated monocycLic structure which has 1 to 5 carbon atoms, contains a further nitrogen atom, oxygen atom or sulphur atom, and is optionally substituted by 1 to 3 alkyl groups, each having 1 to 5 carbon atoms-with the proviso that R¹ does not represent methyla at the same time as R² represents phenyl.

2. (5-Trifluoromethyl-1,3,4-thiadiadiazol-2-yl-oxy)-acetic acid 2-methylpiperidide of the formula 2 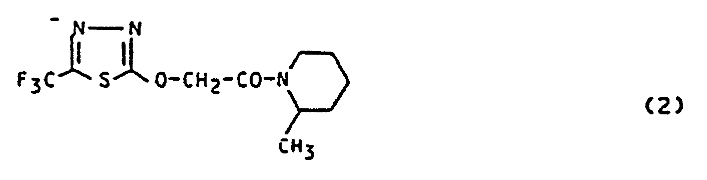 according to Claim 1.

3. (5-Trifluoromethyl-1,3,4-thiadiazol-2-yl-oxy)-acetic acid 2-ethylpiperidide of the formula 40 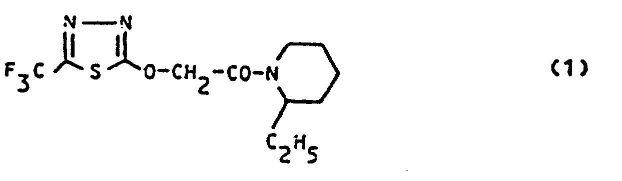 according to Claim 1.

4. (5-Trifluoromethyl-1,3,4-thiadia2ol-2-yl-oxy)-acetic acid N-methyl-N-(2-methyl-phenyl)-amide of the formula 15 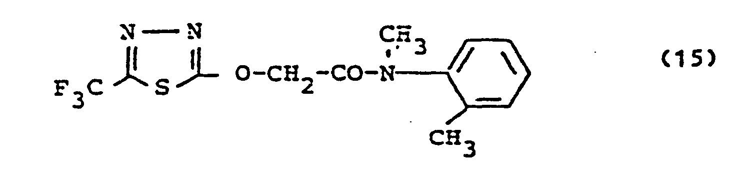 according to Claim 1.

5. (5-Trifluoromethyl-1,3,4-thiadiazol-2-yl-oxy)-acetic acid H-methyl-N-(3-methyl-phenyl)-amide of the formula 18 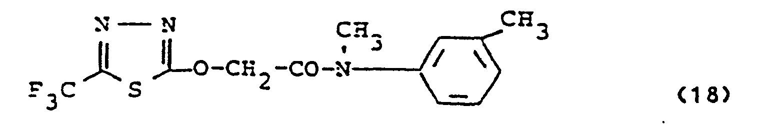 according to Claim 1.

6. (5-Trifluoromethyl-1,3,4-thiadiazol-2-yl-oxy)-acetic acid N,N-diethylamide of the formula 5 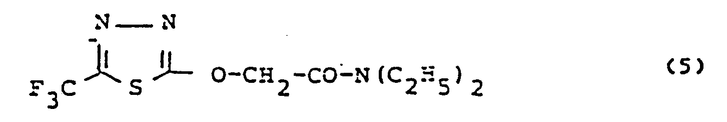 according to Claim 1.

7. (5-Trifluoromethyl-1,3,C-tniazdiazol-2-yl-oxy)-acetic acid N,N-(di-n-propylamide) of the formula 25 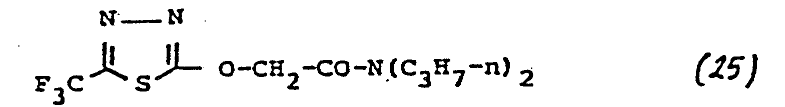 according to Claim 1.

8. Process for the preparation of substituted 5-trifluoromethyl-1,3,4-thiadiazol-2-yl-oxyacetic acid amides of the general formula 1 according to Claim 1, characterised in that hydroxyacetic acid amides of the general formula II 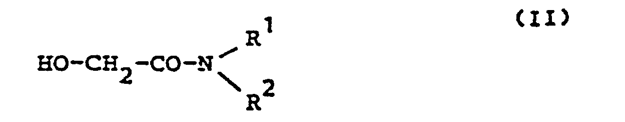 in which
R^{I} and R² have the meaning given above, are reacted with 2-chloro-5-trifluoromethyl-1,3,4-thia-diazole of the formula III 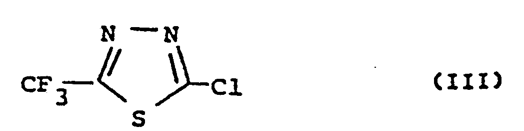 in the presence of an acid acceptor and, if appropriate, using a diluent.

9. Herbicidal agents, characterised in that they contain substituted 5-trifluoromethyl-1,3,4-thiadiazol-2-yl-oxyacetic acid amides of the general formula I according to Claim 1.

10. Use of substituted 5-trifluoromethyl-1,3,4-thia- diazol-2-yl-oxyacetic acid amides of the general formula according to Claim 1, for combating plant growth.

11. Process for the preparation of herbicidal agents, characterised in that substituted 5-trifluoromethyl- 1,3,4-thiadiazol-2-yi-oxyacetic acid amidesof the general formula I according to Claim 1 are mixed with extenders and/or surface-active agents.

## Revendications

1. Amides d'acide 5-trifluorométhyl-1,3,4-thiadiazole-2-yloxyacétique substitués de formule générale 1 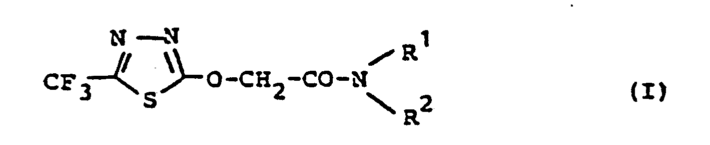 dans laquelle
R¹ et R² sont identiques ou différents et représentent individuellement un groupe alkyle, cyanalkyle, alkoxyalkyle, alkylthioalkyle, alcényle, alcynyle, alkoxy, chacun ayant jusqu'à 10 atomes de carbone, un groupe cycloalkyle ou cycloalcényle ayant chacun jusqu'à 12 atomes de carbone, un groupe aralkyle ayant 1 ou 2 atomes de carbone dans la partie alkyle et 6 ou 10 atomes de carbone dans la partie aryle, qui est éventuellement substituée par un halogène, ou un reste aryle ayant 6 ou 10 atomes de carbone, le reste aryle pouvant être substitué par 1 à 3 atomes d'halogènes, 1 à 3 groupes alkyle ayant chacun 1 à 4 atomes de carbone, un groupe nitro, cyano ou alkoxy ayant 1 à 4 atomes de carbone, ou bien les restes R^{l} et R² forment, conjointement avec l'atome d'azote auquel ils sont liés, un noyau monocyclique ou bicyclique ayant jusqu'à'15 atomes de carbone, éventuellement en partie insaturè et/ou condensé à du benzène, le cas échéant substitué par 1 à 3 groupes alkyle ayant chacun 1 à 5 atomes de carbone, ou bien les restes R¹ et R² forment, conjointement avec l'atome d'azote auquel ils sont liés, un noyau monocyclique ayant 1 à 5 atomes de carbone, éventuellement substitué par 1 à 3 groupes alkyle ayant chacun 1 à 5 atomes de carbone, saturé et comportant un autre atome d'azote, atome d'oxygène ou atome de soufre - sous réserve que R¹ ne soit pas un groupe méthyle en même temps que R est un groupe phényle.

2. 2-méthylpipéridide d'acide (5-trifluoro-méthyl-1,3,4-thiadiazole-2-yloxy)acétique de formule 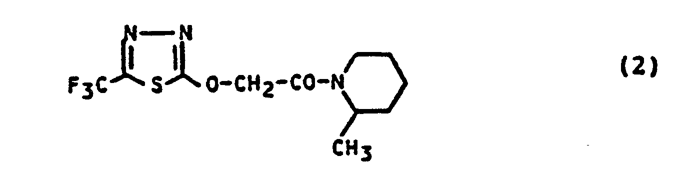 suivant la revendication 1.

3. 2-éthylpipéridide d'acide (5-trifluoro-méthyl-1,3,4-thiadiazole-2-yloxy)acétique de formule 1 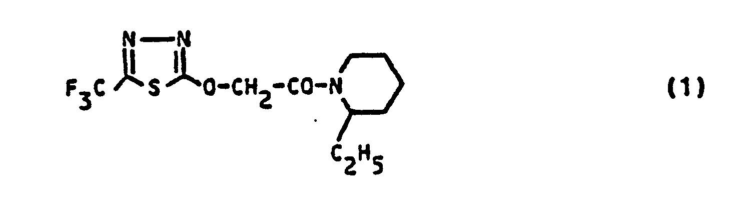 suivant la revendication 1.

4. N-méthyl-N-(2-méthylphényl)amide d'acide (5-trifluorométhyl-1,3,4-thiadiazole-2-yloxy)acétique de formule 15 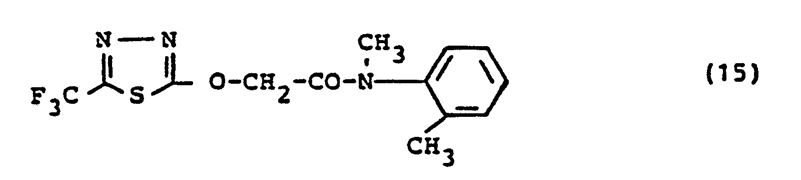 suivant 12 revendication 1.

5. N-méthyl-N-(3-méthylphényl)amide d'acide (5-trifluorométhyl-1,3,4-thiadiazole-2-yloxy)acétique de formule 18 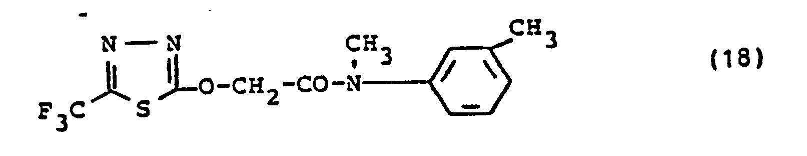 suivant la revendication 1.

6. N,N-diéthylamide d'acide (5-trifluoro-méthyl-1,3,4-thiadiazole-2-yloxy)acétique de formule 5 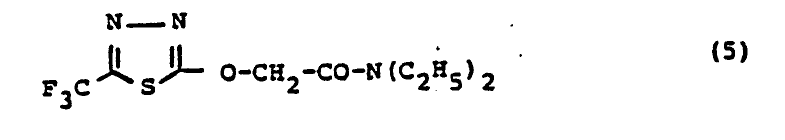 suivant la revendication 1.

7. N,N-(di-n-propylamide) d'acide (5-tri-fluorométhyl-1,3,4-thiadiazole-2-yloxy)acétique de formule 25 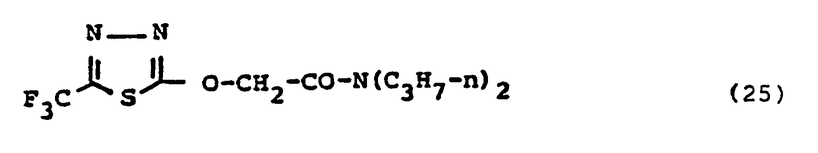 suivant la revendication 1.

8. Procédé de production d'amides d'acide 5-trifluorométhyl-1,3,4-thiadiazole-2-yloxyacétirue substitués de formule générale 1 suivant la revendication 1, caractérisé en ce qu'on fait réagir des amides d'acide hydroxyacétique de formule générale II 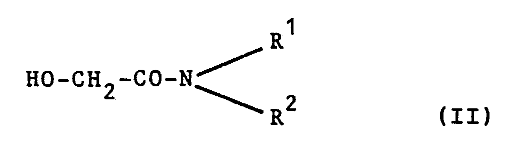 dans laquelle
R^{l} et R² ont la définition indiquée ci-dessus, avec le 2-chloro-5-trifluorométhyl-1,3,4-thiadiazole de formule III 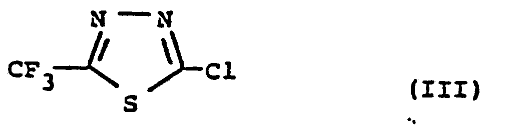 en présence d'un accepteur d'acide et en utilisant éventuellement un diluant.

9. Compositions herbicides, caractérisées par une teneur en amides d'acide 5-trifluorométhyl-1,3,4-thiadiazole-2-yloxyacétique substitués de formule générale 1 suivant la revendication 1.

10. Utilisation d'amides d'acide 5-trifluoro-méthyl-1,3,4-thiadiazole-2-yloxyacétique substitués de formule générale 1 suivant la revendication 1 pour combattre la croissance de plantes.

11. Procédé de préparation de compositions herbicides, caractérisé en ce qu'on mélange des amides d'acide 5-trifluorométhyl-1,3,4-thiadiazole-2-yloxyacé-tique substitués de formule générale I suivant la revendication 1 avec des diluants et/ou des agents tensio-actifs.
